# EUROPEAN PATENT APPLICATION

(11) **EP 4 205 704 A1**
(43) Date of publication of application: **05.07.2023**
(21) Application number: 21949841.7
(22) Date of filing: 20.08.2021
(51) Int. Cl.: A61F 2/24

(54) **STENT LOADING SYSTEM AND METHOD**

(30) Priority: 12.07.2021 CN 202110788788
(71) Applicant: Shanghai Trulive Medtech Co., Ltd, Shanghai 201206 (CN); Jiangsu Trulive Medtech Co., Ltd, Rudong County Nantong Jiangsu 226400 (CN)
(72) Inventor: ZHANG, Haijun, Shanghai 201206 (CN); LIU, Xiang, Shanghai 201206 (CN); WEN, Jing, Shanghai 201206 (CN)
(74) Representative: Argyma
(86) International application number: PCT/CN2021/113656
(87) International publication number: WO 2023/284066

(57) **Abstract**

The invention provides a stent loading system and a method using the same for loading a stent into a sheath. The stent includes a stent body, and an outer periphery of one end of the stent body is provided with a hook. The stent loading system includes a hook straightener for straightening the hook on the stent and a compressor, wherein the stent in which the hook is straightened is reduced in diameter and crimped through the compressor, and the stent crimped is delivered into a preloaded tube; the stent is connected with a delivery system penetrating in the sheath, and the stent in the preloaded tube is delivered into the sheath under the traction of the delivery system.

## Description

### BACKGROUND OF THE INVENTION

### Field of the Invention

The invention relates to the technical field of medical device design, in particular, to a stent loading system and a method using the same.

### Description of the Prior Art

Heart valves are membrane-like structures that can open and close in the organs of humans or certain animals. Everyone's heart has four valves, which are the aortic valve connecting the left ventricle to the aorta, the pulmonary valve connecting the right ventricle to the pulmonary artery, the mitral valve connecting the left atrium to the left ventricle and the tricuspid valve connecting the right atrium to the right ventricle. They all act as one-way valves, so that blood can only flow from one direction to the other and not back.

With social economy development and population aging, the incidence of valvular heart disease has increased significantly. Studies have shown that the incidence of valvular heart disease in the population over 75 years of age is as high as 13.3%. At present, traditional surgical treatment is still the preferred treatment for patients with severe valvular lesions. However, for patients who are older with combined multi-organ diseases, a history of thoracotomy, and poor heart function, the traditional surgery has disadvantages of high risk and mortality and even no access to surgery for some patients.

The tricuspid valve, as an atrioventricular valve of the right heart, has a structure similar to that of the mitral valve, and also includes the valve leaflet, the annulus, the chordae tendineae, the papillary muscle, and the cardiac muscle. Transcatheter tricuspid valve replacement (TTVR) or transcatheter tricuspid valve repair (TTVr) has the advantages of no need for thoracotomy, small trauma, and quick recovery for patients, and has been widely concerned by experts and scholars.

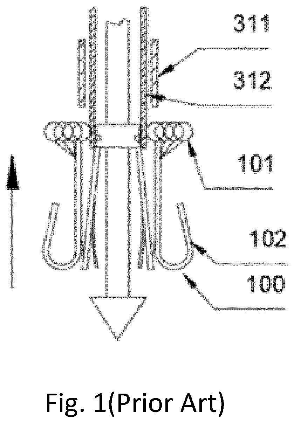

In the prior art, stent designs typically have a flanged construction such as the flange 101 shown in Fig. 1; implantation of the stent requires integral loading of the stent into the sheath 311 at the end of the delivery system 312. The stent 100 is released from the hook 102 in the human body (hooking the valve leaflet first), and the flange 101 is released last, so the flange 101 must be sheathed first from the inflow end to the outflow end (as shown by the arrow in Fig. 1). Generally, the valve stent is compressed in an ice-water bath, but the structure of the flange 101 is in a fluffy state at both normal and ice-water temperatures. Therefore, how to ensure that the flange 101 and the stent 100 are safely and accurately inserted into the sheath has become an urgent problem to be solved.

In addition, the existing stent design has several hooks 102 in the ventricular segment (as shown in Fig. 1, a structure of hooking the valve leaflet shapes like a hook). During loading, the hook 102 needs to be straightened to facilitate loading without damaging the structure of the stent 100. In order to minimize the artificial damage in the process of straightening the hook 102, it is necessary to design a tool to straighten the hook 102 neatly at one time.

### SUMMARY OF THE INVENTION

In view of the problems in the background, the invention provides a stent loading system, which includes:
a hook straightener, used for straightening at least one hook on the stent;
a compressor, wherein the stent on which the at least one hook is straightened is reduced in diameter and crimped through the compressor, and the stent crimped is delivered into a preloaded tube;
the stent is connected with a delivery system penetrating in the sheath, and the stent in the preloaded tube is delivered into the sheath under the traction of the delivery system.

In some embodiments, the system further includes a reducer, wherein the reducer has a reducer section with an inner diameter decreasing gradually, the reducer is disposed between the preloaded tube and the sheath, and the stent in the preloaded tube is delivered into the sheath after being further reduced in diameter through the reducer section.

In some embodiments, an outer periphery of the other end of the stent is provided with a flange portion; the reducer section has a large-diameter end butted and communicated with the preloaded tube, and has a small-diameter end butted and communicated with the sheath, an inner diameter of the small-diameter end being approximately no greater than an inner diameter of the sheath; When the stent is reduced in diameter when passing through the small-diameter end of the reducer to make the diameter smaller than the inner diameter of the sheath.

In some embodiments, the hook straightener includes:
a base, having a first channel with openings at both ends in an axial direction, wherein one end of the first channel is an inlet end, the other end is an outlet end, and an end surface of the inlet end is provided with at least one hook groove; the stent is loaded in the first channel, and one end of the stent provided with the at least one hook is located outside the inlet end, the at least one hook extending into the at least one hook groove;
a propeller, having a second channel with the opening at one end in the axial direction, a propulsion column being coaxially disposed in the second channel;
wherein the base is mounted movably in the second channel from the opening of the second channel in the axial direction, and an end surface of the propulsion column abuts against an end surface of the stent provided with the at least one hook; the base moves toward the propeller, and the propulsion column moves the stent toward the outlet end while the at least one hook being straightened between an inner wall of the first channel and an outer wall of the propulsion column.

In some embodiments, an outer wall of the base is connected with an inner wall of the second channel by threads, and the base is rotated to realize axial movement with the propeller;
or an outer wall of the base is connected movably with an inner wall of the second channel in the axial direction by a rail assembly, and the base is pulled in the axial direction to realize axial movement with the propeller.

In some embodiments, an end surface of the propulsion column facing toward one end of the base is provided with an inner recess, and a hook straightening section is formed around the inner recess; an end portion of the stent extends into the inner recess, and the hook straightening section is placed between the stent and the at least one hook.

In some embodiments, the system further includes at least one protective tube, wherein before the stent is crimped by the compressor, the at least one protective tube penetrates through a center of the stent in the axial direction.

In some embodiments, the system includes a plurality of protective tubes with diameters gradually decreasing from large to small, wherein first the protective tubes with a large diameter are selected to penetrate through in the stent for crimping, and then the protective tubes with a smaller diameter are gradually selected to penetrate through in the stent for crimping.

In some embodiments, both ends of the reducer section are coaxially provided with a preloaded tube mounting section and a sheath mounting section respectively; an inner diameter of the preloaded tube mounting section is equal to an inner diameter of the large-diameter end of the reducer section, and an inner diameter of the sheath mounting section is not smaller than an inner diameter of the small-diameter end of the reducer section; the preloaded tube is mounted in the preloaded tube mounting section, and the sheath is mounted in the sheath mounting section.

In some embodiments, the reducer is composed of two semi-reducers in a radial direction, and the two semi-reducers are fixed by connecting members at both ends after being assembled.

In some embodiments, a side wall of the reducer is provided with an adjustment port, and the adjustment port is extended parallel to the axial direction of the reducer; when the stent passes through the reducer, the flange portion is stuck, and the stent is adjusted by penetrating through the adjustment port with a toggling sheet.

In some embodiments, a delivery portion of the delivery system is mounted movably in the sheath, and one end of the delivery portion extends out of the outer sheath to be connected with the stent crimped; then the preloaded tube is sleeved on the stent, followed by mounting the reducer onto the preloaded tube and the outer sheath; the delivery portion moves to pull the stent out of the protective tube for entering into the sheath after passing through the reducer.

In some embodiments, the delivery portion is provided with a hanging hook base, and the stent crimped is hung to the hanging hook base to realize connection through a hanging hook on one end thereof.

The invention further provides a stent loading method, which uses the stent loading system mentioned above and includes steps of:
S1, placing the stent to be loaded into chilled saline;
S2, straightening the at least one hook on the stent by a hook straightener;
S3, reducing and crimping the stent in diameter to a target size by the compressor;
S4, connecting the stent crimped with the delivery system;
S5, sleeving the stent by using the preloaded tube;
S6, mounting the reducer onto the preloaded tube and the sheath;
S7, moving the delivery portion of the delivery system relative to the sheath to pull the stent out of the protective tube for entering into the sheath after passing through the reducer.

In some embodiments, the step S1 further includes setting a temperature of the chilled saline to be lower than 5°C.

In some embodiments, the step S3 further includes: first selecting the protective tubes with a larger diameter to penetrate through at the center of the stent, and then compressing the stent by using the compressor; after compression, selecting the protective tubes with a relatively smaller diameter to penetrate through at the center of the stent, and then compressing the stent by using the compressor; by analogy, selecting the protective tubes with a diameter decreasing gradually to penetrate through in the stent, and compressing the stent by using the compressor repeatedly until the stent is compressed to the target size in the radial direction.

In some embodiments, the step S5 further includes: first sleeving the stent with the preloaded tube, then extracting the protective tubes, and continuously propelling the preloaded tube until flanges on the stent are fully constrained.

In some embodiments, the step S7 further includes: removing the connecting member on the reducer after the flange portion of the stent moves into the sheath completely to cancel a crimping force exerted on the stent in the radial direction; then continuously pulling the stent to move until the stent is fully moved into the sheath.

In some embodiments, the step S7 further includes: observing if the flange portion is stuck during the process of pulling the stent by the delivery portion, and if so, adjusting the flange portion by extending a toggling sheet into the reducer after penetrating the toggling sheet through the adjustment port on the reducer.

Due to the above technical solutions, the invention has the following advantages and beneficial effects as compared to the prior art:
For the stent loading device provided by the invention, first, the straightening of the hook is achieved through the provision of the hook straightener, so as to prepare for the subsequent crimping of the stent, and also reduce the risk of injury caused by artificial straightening; second, the provision of the reducer allows the stent to enter the sheath smoothly, wherein specifically, during delivery, the end of the stent provided with the flange portion enters the reducer first, and a compression tool is used to further compress the reducer in the radial direction when passing through the reducer section whose inner diameter gradually decreases for making the size of the small-diameter end smaller than the inner diameter of the sheath, so that the radial dimension of the flange coming out of the small-diameter end must be smaller than the inner diameter of the sheath and will not touch the end of the sheath, thereby ensuring that the stent can be smoothly delivered into the sheath and the shape of the flange portion will not be folded or bent when being inserted into the sheath.

### BRIEF DESCRIPTION OF THE DRAWINGS

The above and other features and advantages of the invention can be more clearly understood through the following detailed description in conjunction with the accompanying drawings , wherein:
- Fig.1: is a view showing the process of mounting a stent into a sheath according to the prior art;
- Fig. 2: is a view of the stent according to the invention;
- Fig. 3: is a cross-sectional view of a base according to the invention;
- Fig. 4: is a cross-sectional view of the propeller according to the invention;
- Fig. 5: is a view showing the process of straightening a hook according to the invention;
- Fig. 6: is a structural view of the stent after the hook is straightened according to the invention;
- Fig. 7: is a view of compressing the stent by a compressor according to the invention;
- Fig. 8: is a structural view of the stent after being compressed according to the invention;
- Fig. 9: is a cross-sectional view of a reducer according to the invention;
- Fig. 10: is a view showing the disassembly of the reducer according to the invention;
- Fig. 11: is a view showing the working principle of the reducer according to the invention;
- Fig. 12: is a view after the stent is connected with a delivery system according to the invention;
- Fig. 13: is a view of mounting the stent into a preloaded tube according to the invention;
- Fig. 14: is a view after the reducer is connected with the preloaded tube and the sheath according to the invention;
- Fig. 15: is a view after the stent is loaded into the sheath according to the invention;
- Fig. 16: is a flow chart of a method for loading the stent according to the invention.

### DESCRIPTION OF THE PREFERRED EMBODIMENTS

The invention will be described in more detail hereinafter with reference to the accompanying drawings showing embodiments of the invention. However, the invention may be embodied in many different forms and should not be construed as limited to the embodiments set forth herein. Rather, these embodiments are provided so that the disclosure will be thorough and complete, and will fully convey the scope of the invention to those skilled in the art. In the drawings, the size and relative sizes of layers and regions may be exaggerated for clarity.

It should be noted that all the directional indications (such as up, down, left, right, front, back) in the embodiments of the present invention are only used to explain the relative positional relationship, motion situation, and the like between the components under a specific attitude (as shown in the drawings), and if the specific attitude changes, then the directional indications also change accordingly.

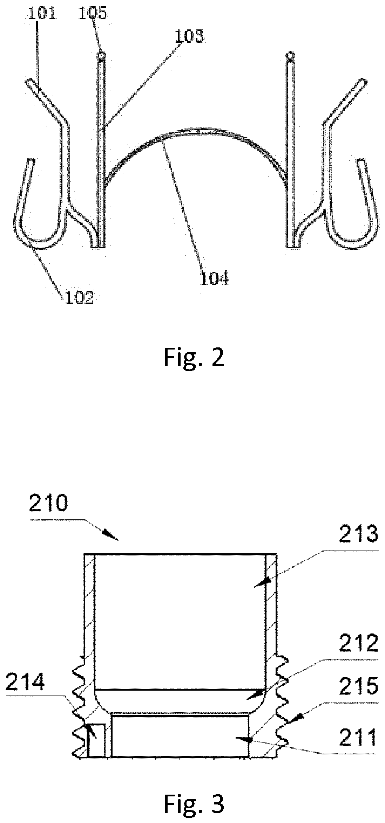

The invention provides a stent loading system and a method using the same in order to load the stent into a sheath at an end portion of a delivery system, which is mainly used for loading the stent provided with a flange portion and a hook such as a heart valve stent shown in Fig. 2. Specifically, the stent includes a stent body 103, wherein an outer periphery of one end of the stent body 103 is provided with one or more hooks 102, and an outer periphery of the other end of the stent body is provided with the flange portion 101; the hook 102 is of a hook-shaped structure, the flange portion 101 is a structure extending away from the stent body 103, and a center of the stent body 103 is also provided with a valve 104.

The invention provides a stent loading system and a method using the same, so as to solve the problem of pre-straightening the hook in the prior art and the problem of safety and accuracy when the flange portion and the stent enter the sheath.

Further explanations are given below with regard to specific embodiments:

### Embodiment 1

With reference to Figs. 2 to 15, the invention provides a stent loading system, including a hook straightener, a compressor 3, and a reducer 4. The hook straightener is used to straighten the hook 102 on the stent 100; the stent in which the hook 102 is straightened is reduced in diameter and crimped through the compressor 3, and the stent crimped is delivered into a preloaded tube 5; the stent 100 is connected with a delivery system 311 penetrating in the sheath, and the stent in the preloaded tube 5 is delivered into the sheath 311 under the traction of the delivery system. For the stent loading device provided by the invention, the hook is straightened through the provision of the hook straightener, so as to prepare for the crimping of the subsequent stent, and also reduces the risk of injury caused by artificial straightening.

In the embodiment, the system further includes the reducer 4. The reducer 4 has a reducer section 410 with an inner diameter decreasing gradually; the reducer section 410 has a large-diameter end butted and communicated with the preloaded tube 5, and has a small-diameter end butted and communicated with the sheath 311, an inner diameter of the small-diameter end being no greater than an inner diameter of the sheath 311; the stent 100 is connected with the delivery system penetrating in the sheath 311, and the stent in the preloaded tube 5 is delivered into the sheath 311 through the reducer section 410 under the traction of the delivery system.

For the stent loading device provided by the invention, first, the straightening of the hook is achieved through the provision of the hook straightener, so as to prepare for the subsequent crimping of the stent, and also reduce the risk of injury caused by artificial straightening; second, the provision of the reducer 4 allows the stent to enter the sheath 311 smoothly, wherein specifically, during delivery, the end of the stent 100 provided with the flange portion 101 enters the reducer 4 first, and the flange portion 101 is further compressed when passing through the reducer section 410 whose inner diameter gradually decreases for making the size smaller than the inner diameter of the sheath, so that the radial dimensions of the stent coming out of the small-diameter end and the flange portion must be smaller than the inner diameter of the sheath and will not touch the end of the sheath, thereby ensuring that the stent can be smoothly delivered into the sheath and the shape of the flange portion 101 will not be folded or bent when being inserted into the sheath.

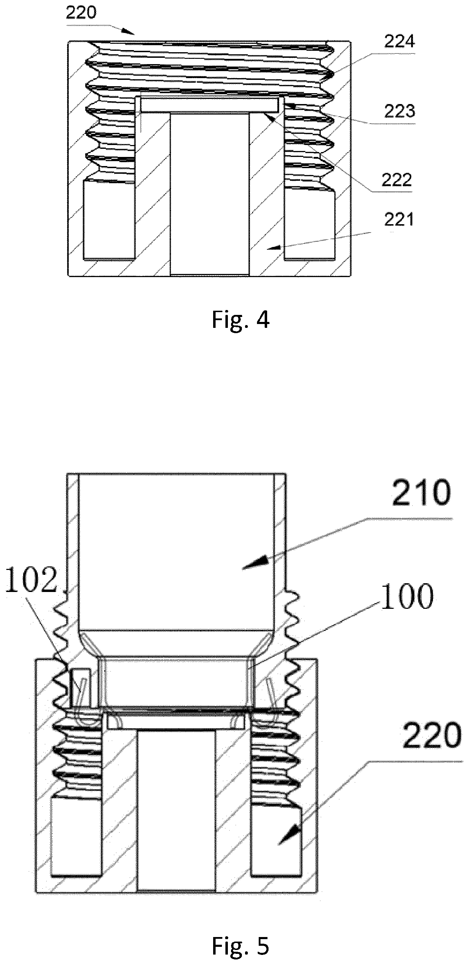

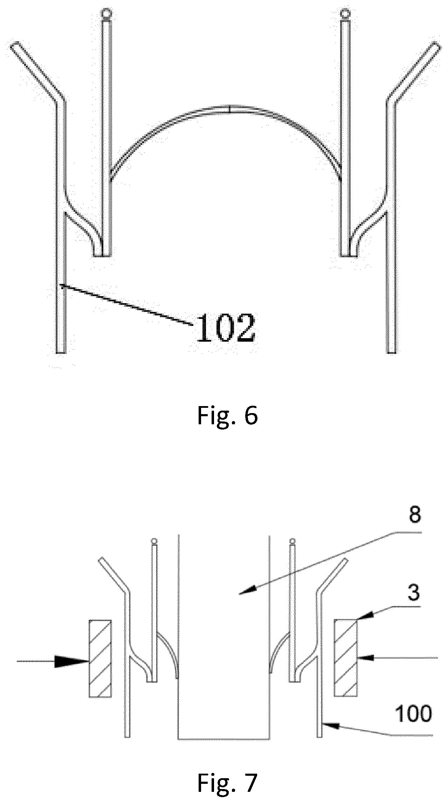

In the embodiment, with reference to Figs. 3 to 5, the hook straightener includes a base 210 and a propeller 220, wherein the base 210 is provided with a first channel with openings at both ends in an axial direction with one end being an inlet end and the other end being an outlet end, an end surface of the inlet end is provided with a hook groove 214, and the number of hook grooves 214 is consistent with the number of the hooks 102 on the stent, which is not limited here; the stent 100 is loaded in the first channel, an end of the stent provided with the hook is located outside the inlet end, so that the hook 102 extends into the hook groove, as shown in Fig. 5; the propeller 220 is provided with a second channel with openings at one end in the axial direction (the opening is configured towards the base 210 ), and a propulsion column 221 is coaxially arranged in the second channel; the base 210 is movably mounted in the second channel in the axial direction from the opening of the second channel, and an end surface of the propulsion column 221 abuts against an end surface of the end of the stent 100 provided with the hook 102; when in use, the base 210 is moved towards the propeller 220, and the propulsion column 221 moves the stent 100 to the outlet end of the first channel while straightening the hook between an inner wall of the first channel and an outer wall of the propulsion column 221, so as to form the stent structure shown in Fig. 6. The hook straightener provided by the embodiment finish straightening all the hooks 102 at one time, which has a simple structure and is convenient in operation.

Further, a first threaded portion 215 is provided on an outer wall surface of one end of the base 210 facing the propeller 220, a second threaded portion 224 is provided on an inner wall surface of the second channel of the propeller 220, the base 210 is in threaded connection with the propeller 220 through the first threaded portion 215 and the second threaded portion 224 while realizing axial movement through relative rotation. The threaded connection has the advantages of convenient operation and stable movement. In other words, in other embodiments, the axial movement between the base 210 and the propeller 220 is not limited to the above, and can also be adjusted according to specific conditions, for example, by means of slide rails, etc., which is not limited here.

Further, with reference to Figs. 3 and 5, the first channel of the base 210 is divided into an inlet section 211, a transition section 212, and an outlet section 213, wherein the inner diameters of the transition section 212 and the outlet section 213 are larger than an inner diameter of the inlet section 211, the inner diameter of the inlet section 211 matches the size of the stent body 103, and a side wall of the transition section 212 is arc-shaped; initially, the flange portion 101 of the stent is located in the transition section 212, and during the straightening process, the stent is gradually pushed from the inlet section 211 through the transition section 212 to the outlet section 213 and is delivered from an end of the outlet section 213. In the embodiment, the segmental design of the first channel ensures the stability of the movement of the stent during delivery and prevents the stent from being damaged.

Further, with reference to Figs. 4 and 5, an end surface of one end of the propulsion column 220 facing the base 210 is provided with an inner recess, and a periphery of the inner recess constitutes a hook straightening section 223; an end of the stent extends into the inner recess, the hook straightening section is placed between the stent and the hook 102, and the straightening of the hook 102 is realized by the cooperation between the hook straightening section 223 and the inner wall of the first channel. In the embodiment, a lower end of the stent is stuck in the inner recess through the provision of the inner recess, thereby ensuring the stability of the stent during the straightening process of the hook 102; of course, the provision of the inner recess can also be omitted in other embodiments, and there is no limitation here.

In the embodiment, the stent in which the hook 102 is straightened is shrunk in the radial direction through the compressor 3; the compressor may directly adopt a stent compressor in the prior art, and there is no limitation here.

Further, the stent loading device further includes at least one protective tube, wherein before the stent 100 is crimped by the compressor, the protective tube is mounted at an axial center of the stent 100, and the protective tube stretches the valve 104 at a center of the stent. In the embodiment, through the provision of the protective tube, the valve at the center of the stent is protected, and the valve is prevented from being squeezed and damaged at the center during the compression process of the stent.

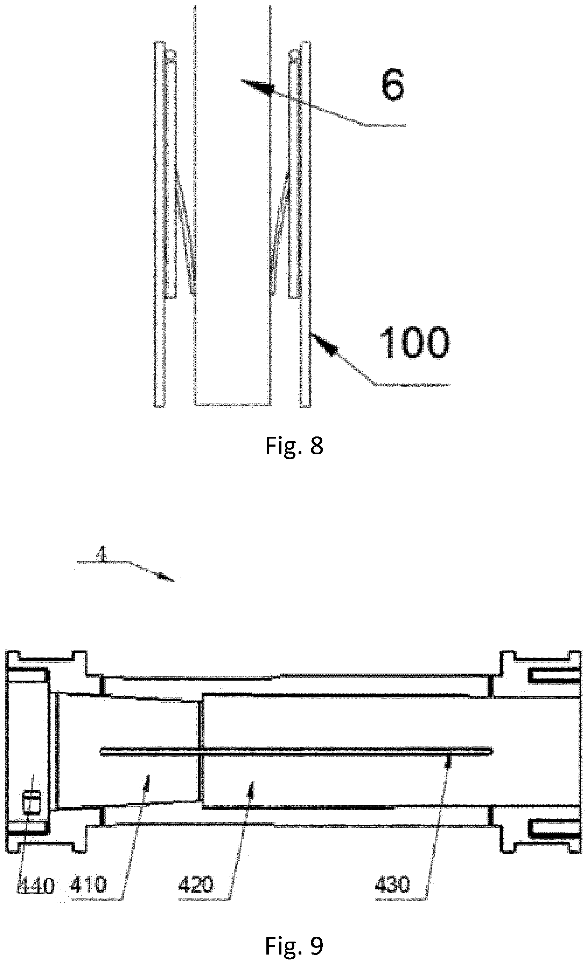

Further, in the embodiment, the stent is compressed multiple times in the radial direction by the compressor to achieve a gradual reduction in the radial direction of the stent until the stent is compressed to a target size as shown in Fig. 8, which is beneficial to ensure that the stent is not damaged. Correspondingly, a plurality of protective tubes with diameters gradually decreasing from large to small are required, wherein firstly, the protective tube 8 with a larger diameter is selected to be threaded in the stent 100 for crimping, and then the protective tube 6 with a smaller diameter is gradually selected to be threaded in the stent for crimping.

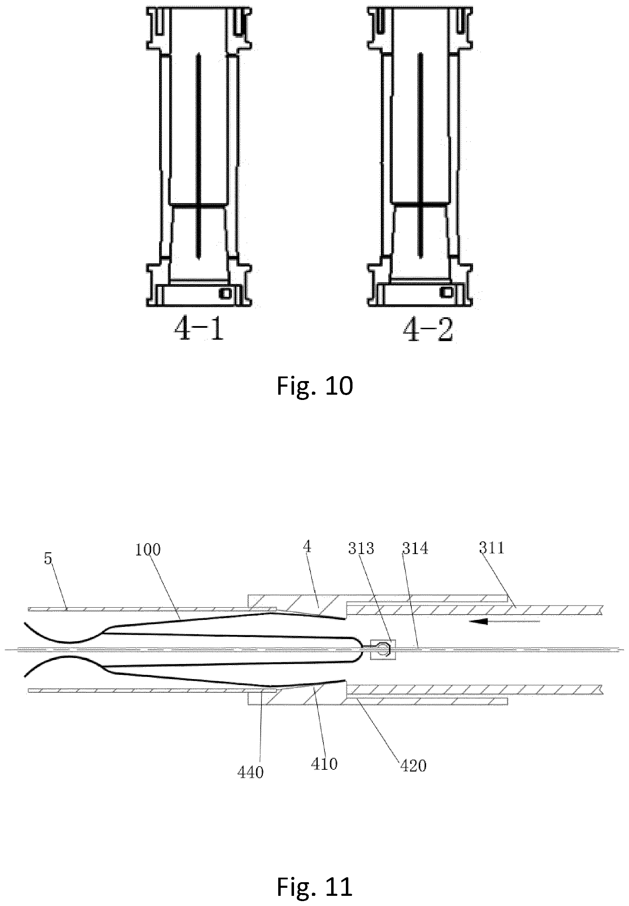

In the embodiment, with reference to Figs. 9 and 11, the reducer 4 is composed of a preloaded tube mounting section 440, a reducer section 410, and a sheath mounting section 430 connected coaxially in sequence; an inner diameter of the preloaded tube mounting section 440 is equal to an inner diameter of the large-diameter end of the reducer section 410, and an inner diameter of the sheath mounting section 430 is larger than an inner diameter of the small-diameter end of the reducer section 410; the preloaded tube 5 is mounted in the preloaded tube mounting section 440, and the sheath 311 is mounted in the sheath mounting section 430. In the embodiment, through the provisions of the preloaded tube mounting section 440 and the sheath mounting section 430, the preloaded tube 5 and the sheath 311 are arranged coaxially at both ends of the reducer section 410, so that the stent 100 may be delivered accurately.

In the embodiment, as shown in Fig. 10, the reducer 4 is composed of two semi-reducers in a radial direction, which are respectively a left semi-reducer 4-1 and a right semi-reducer 4-2; when in use, the left semi-reducer 4-1 and the right semi-reducer 4-2 are arranged on outer walls of the preloaded tube 5 and the sheath 311, so that the preloaded tube 5 and the sheath 311 are respectively placed in the preloaded tube mounting section 440 and the sheath mounting section 430; then, two ends of the left semi-reducer 4-1 and the right semi-reducer 4-2 are respectively fixed by connectors, and the connectors may be realized by structures such as snap rings, which are not limited here.

Further, when in use, after the flange portion 101 of the stent moves into the sheath, the connectors may be removed, thereby removing the radial crimping force on the stent, so as to avoid fatigue or valve damage caused by excessive compression of the stent.

In the embodiment, with reference to Fig. 9 again, an adjustment port 430 is provided on a side wall of the reducer 4, and the adjustment port 430 is extended parallel to the axial direction of the reducer 4 and spans three areas of the preloaded tube mounting sections 440, the reducer section 410 and the sheath mounting section 430. In the embodiment, through the provision of the adjustment port 430, when the stent 100 passes through the reducer 4, if the flange portion is stuck, a toggling sheet is inserted through the adjustment port 430 into the reducer 4 to adjust the stent so that the stent may be smoothly delivered into the sheath.

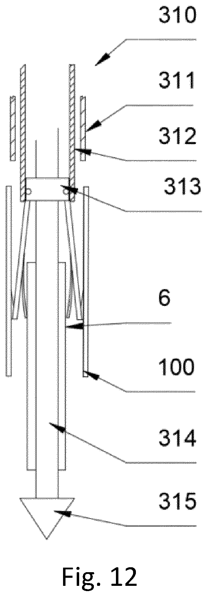

In the embodiment, the delivery portion of the delivery system 310 may be movably mounted in the sheath 311, and one end of the delivery portion extends out of the sheath 311 to be connected with the stent that has been crimped, as shown in Fig. 12; specifically, the delivery portion of the delivery system 310 includes an inner sheath 312 and a guiding wire 314 connected with the inner sheath 312, wherein one end of the guiding wire 314 is connected with the inner sheath 312, and the other end of the guiding wire passes through the protective tube 6 at the center of the stent to be connected with a Tip head, wherein the Tip head is mainly used to protect the stent during the implantation process, and the delivery system does not damage the original tissue, such as blood vessel avoidance, valve annulus leaflets when crossing the valve, and the function of guiding the delivery system to implant, etc.; the inner sheath 312 is hung with a hanging hook base 313, and the stent that has been crimped is hung to the hanging hook base 313 for connection through the hanging hook 105 at one end. In other words, in other embodiments, the connection way between the stent and the delivery portion is not limited to the above, and can also be adjusted according to specific conditions.

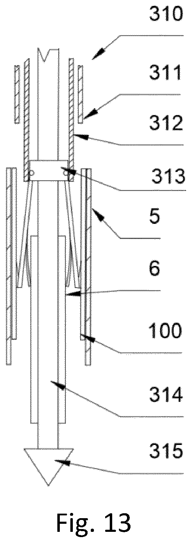

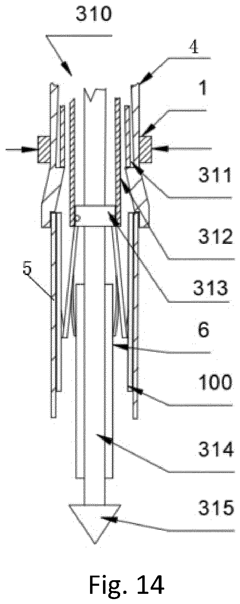

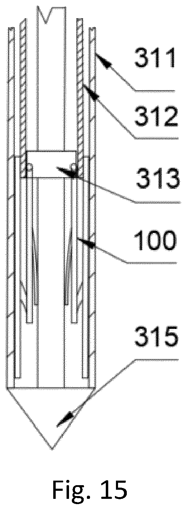

Then, the preloaded tube 5 is sleeved onto the stent 100, as shown in Fig. 13; and then, the reducer 4 is mounted onto the preloaded tube 5 and the outer sheath 311, as shown in Fig. 14; the delivery portion moves to pull the stent 100 out of the protective tube to enter the sheath 311 through the reducer 4, as shown in Fig. 15.

### Embodiment 2

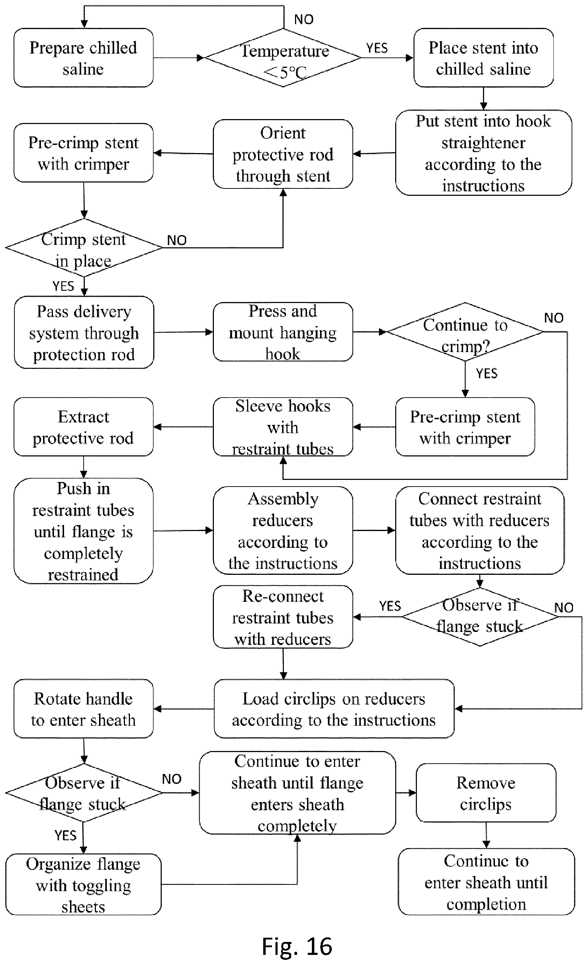

With reference to Fig. 16, the embodiment further provides a stent loading method, which adopts the stent loading system as mentioned in Embodiment 1, wherein the specific steps of the stent loading method are as follows:
S 1, placing the stent to be loaded into chilled saline;
   specifically, the stent to be loaded is placed in chilled saline for loading, so as to make the stent fluffy, facilitating operations such as compression; further, a temperature of the chilled saline is less than 5° C, and when the temperature is not satisfied, preparation is performed again until the temperature meets the requirements; preferably, the temperature of the chilled saline is 0-5 °C.
S2, straightening the hook on the stent by a hook straightener;
   the specific structure of the hook straightener and the way of straightening are described with reference to Embodiment 1, and will not be repeated here.
S3, reducing and crimping the stent is in diameter to a target size by the compressor;
   before the compressor is used, a protective tube is provided at the center of the stent in the axial direction; first, preferably the protective tube with a larger diameter is selected to be provided on the stent for being crimped through the compressor; then, the protective tubes with reduced diameters are selected gradually to be provided on the stent for being crimped through the compressor; the above operations are repeated several times until the stent is compressed to the target size.
S4, connecting the stent crimped with the delivery system;
   specifically, with reference to Fig. 12, the guiding wire 314 of the delivery system is introduced through the protective tube 6 at the center of the stent 100 while hanging the stent 100 to the hanging hook base 313 on the inner sheath 312 for connection through the hanging hooks of the stent 100 at the ends.
S5, sleeving the stent with the preloaded tube;
   specifically, with reference to Fig. 13, the preloaded tube 5 is sleeved on the stent 100, then the protective tube 6 is removed, and the preloaded tube 5 is continued to be pushed until the flange on the stent 100 is completely restricted, so that the stent is completely restricted in the preloaded tube 5.
S6, mounting the reducer onto the preloaded tube and the sheath;
   specifically, with reference to Fig. 14, the reducer 4 is composed of the left semi-reducer 4-1 and the right semi-reducer 4-2, and the left semi-reducer and the right semi-reducer are provided around the out walls of the preloaded tube 5 and the sheath 311 with both ends then fixed by the connectors respectively.
S7, the delivery portion of the delivery system is moved relative to the sheath to pull the stent out of the protective tube for entering into the sheath after passing through the reducer.

Specifically, after the reducer 4 is mounted, the delivery portion drives the stent 100 to move, and then the flange portion 101 of the stent 100 extends out of the preloaded tube 5 and is radially shrunk when passing through the reducer section firstly and then smoothly enters the sheath 311;
after the flange portion 101 of the stent moves into the sheath 311 completely, the connectors on the reducer 4 may be removed, thereby removing the radial crimping force on the stent, so as to avoid fatigue or valve damage caused by excessive compression of the stent. Then, the stent 100 continues to move toward the sheath until the stent is completely moved into the sheath 311, as shown in Fig. 15.

Further, whether the flange portion is stuck is observed during the process of pulling the stent by the delivery portion, and the flange portion is adjusted by extending the toggling sheet into the reducer 4 after penetrating the toggling sheet through the adjustment port on the reducer 4 if the flange portion is stuck.

The stent loading method provided in the embodiment ensures the smooth insertion of the flange portion of the stent into the sheath, and ensures the correctness and safety of the stent loading.

Those skilled in the art will appreciate that the present invention may be embodied in many other specific forms without departing from its own spirit or scope. Although examples of embodiments of the present invention have been described, it should be understood that the present invention should not be limited to these examples. Changes and modifications may be made by those skilled in the art within the spirit and scope of the invention as defined by the appended claims.

## Claims

1. A stent loading system, used for loading a stent into a sheath, wherein the stent comprises a stent body, an outer periphery of one end of the stent body is provided with at least one hook, and the stent loading system comprises:
a hook straightener, used for straightening the at least one hook on the stent; and
a compressor, wherein the stent on which the at least one hook is straightened is reduced in diameter and crimped through the compressor, and the stent crimped is delivered into a preloaded tube;
wherein the stent is connected with a delivery system penetrating in the sheath, and the stent in the preloaded tube is delivered into the sheath under the traction of the delivery system.

2. The stent loading system according to claim 1, further comprising a reducer having a reducer section with an inner diameter decreasing gradually, wherein the reducer is disposed between the preloaded tube and the sheath, and the stent in the preloaded tube is delivered into the sheath after being further reduced in diameter through the reducer section.

3. The stent loading system according to claim 2, wherein an outer periphery of the other end of the stent is provided with a flange portion;
a large-diameter end of the reducer section is butted and communicated with the preloaded tube, and a small-diameter end thereof is butted and communicated with the sheath;
an inner diameter of the small-diameter end is approximately no greater than an inner diameter of the sheath;
the stent is reduced in diameter when passing through the small-diameter end of the reducer, such that a diameter of the stent is smaller than the inner diameter of the sheath.

4. The stent loading system according to claim 1 or 3, wherein the hook straightener comprises:
a base having a first channel with openings at both ends in an axial direction, wherein one end of the first channel is an inlet end, the other end is an outlet end, and an end surface of the inlet end is provided with at least one hook groove;
the stent is loaded in the first channel, one end of the stent is provided with the at least one hook is located outside the inlet end, and the at least one hook is extending into the at least one hook groove;
a propeller having a second channel with an opening at one end in the axial direction, and a propulsion column is coaxially disposed in the second channel;
wherein the base is mounted movably in the second channel from the opening of the second channel in the axial direction, and an end surface of the propulsion column abuts against an end surface of the stent provided with the at least one hook; the base moves toward the propeller, and the propulsion column moves the stent toward the outlet end while the at least one hook being straightened between an inner wall of the first channel and an outer wall of the propulsion column.

5. The stent loading system according to claim 4, wherein an outer wall of the base is connected with an inner wall of the second channel by threads, such that the base can be rotated to realize axial movement with the propeller; or
an outer wall of the base is connected movably with an inner wall of the second channel in the axial direction by a rail assembly, such that the base can be pulled in the axial direction to realize axial movement with the propeller.

6. The stent loading system according to claim 4, wherein the end surface of the propulsion column facing toward one end of the base is provided with an inner recess, and a hook straightening section is formed around the inner recess;
an end portion of the stent extends into the inner recess, and the hook straightening section is placed between the stent and the at least one hook.

7. The stent loading system according to claim 1, further comprising at least one protective tube, wherein the at least one protective tube penetrates through a center of the stent in the axial direction before the stent is crimped by the compressor.

8. The stent loading system according to claim 7, comprising a plurality of protective tubes with diameters gradually decreasing from large to small, wherein at first, the protective tubes with a large diameter are selected to penetrate through in the stent for crimping, and then the protective tubes with a smaller diameter are gradually selected to penetrate through in the stent for crimping.

9. The stent loading system according to claim 3, wherein both ends of the reducer section are coaxially provided with a preloaded tube mounting section and a sheath mounting section respectively, an inner diameter of the preloaded tube mounting section is equal to an inner diameter of the large-diameter end of the reducer section, and an inner diameter of the sheath mounting section is not smaller than an inner diameter of the small-diameter end of the reducer section;
the preloaded tube is mounted in the preloaded tube mounting section, and the sheath is mounted in the sheath mounting section.

10. The stent loading system according to claim 9, wherein the reducer is composed of two semi-reducers in a radial direction, and the two semi-reducers are fixed by connecting members at both ends after being assembled.

11. The stent loading system according to claim 3, wherein a side wall of the reducer is provided with an adjustment port, and the adjustment port is extended parallel to the axial direction of the reducer;
when the stent passes through the reducer, the flange portion is stuck, and the stent can be adjusted by penetrating through the adjustment port with a toggling sheet.

12. The stent loading system according to claim 1, wherein a delivery portion of the delivery system is mounted movably in the sheath, and one end of the delivery portion extends out of the outer sheath to be connected with the stent crimped; then the preloaded tube is sleeved on the stent, followed by mounting the reducer onto the preloaded tube and the outer sheath; the delivery portion moves to pull the stent out of the protective tube for entering into the sheath after passing through the reducer.

13. The stent loading system according to claim 12, wherein the delivery portion is provided with a hanging hook base, and through a hanging hook on one end of the stent, the stent crimped is hung to the hanging hook base to realize connection.

14. A stent loading method, using the stent loading system according to any one of claims 1 to 13, comprising steps of:
S 1, placing the stent to be loaded into chilled saline;
S2, straightening the at least one hook on the stent by the hook straightener;
S3, reducing and crimping the stent in diameter to a target size by the compressor;
S4, connecting the stent crimped with the delivery system;
S5, sleeving the stent by using the preloaded tube;
S6, mounting the reducer onto the preloaded tube and the sheath;
S7, moving the delivery portion of the delivery system relative to the sheath to pull the stent out of the protective tube for entering into the sheath after passing through the reducer.

15. The stent loading method according to claim 14, wherein the step S1 further comprises setting a temperature of the chilled saline to be lower than 5°C.

16. The stent loading method according to claim 14, wherein the step S3 further comprises:
at first, selecting the protective tubes with a larger diameter to penetrate through at the center of the stent, and then compressing the stent by using the compressor;
after compression, selecting the protective tubes with a relatively smaller diameter to penetrate through at the center of the stent, and then compressing the stent by using the compressor; and
by analogy, selecting the protective tubes with a diameter decreasing gradually to penetrate through in the stent, and compressing the stent by using the compressor repeatedly until the stent is compressed to the target size in the radial direction.

17. The stent loading method according to claim 14, wherein the step S5 further comprises:
at first, sleeving the stent with the preloaded tube, then extracting the protective tubes, and continuously propelling the preloaded tube until flanges on the stent are fully constrained.

18. The stent loading method according to claim 14, wherein the step S7 further comprises:
removing the connecting member on the reducer after the flange portion of the stent moves into the sheath completely to cancel a crimping force exerted on the stent in the radial direction; and then
continuously pulling the stent to move until the stent is fully moved into the sheath.

19. The stent loading method according to claim 14, wherein the step S7 further comprises:
observing if the flange portion is stuck during the process of pulling the stent by the delivery portion, and if so, adjusting the flange portion by extending a toggling sheet into the reducer after penetrating the toggling sheet through the adjustment port on the reducer.
